# EUROPEAN PATENT APPLICATION

(11) **EP 4 431 089 A1**
(43) Date of publication of application: **18.09.2024**
(21) Application number: 22894657.0
(22) Date of filing: 07.11.2022
(51) Int. Cl.: A61K 31/05, A61K 36/898, A61K 36/66, A61K 36/258, A61K 47/34, A61K 47/36, A61K 47/10, A61K 47/58, A61L 26/00, A61P 17/02

(54) **CANNABIDIOL-CONTAINING HEMOSTATIC COMPOSITION AND APPLICATION THEREOF AS LIQUID BANDAGE**

(30) Priority: 17.11.2021 CN 202111363601
(71) Applicant: Suzhou Qinglan Biomedical Technology Co., Ltd., Suzhou, Jiangsu 215123 (CN)
(72) Inventor: ZHAO, Tian, Suzhou, Jiangsu 215123 (CN); ZHOU, Yanli, Suzhou, Jiangsu 215123 (CN)
(74) Representative: HGF
(86) International application number: PCT/CN2022/130332
(87) International publication number: WO 2023/088120

(57) **Abstract**

A cannabidiol-containing hemostatic composition, comprising, in parts by mass, 0.05 to 2 parts of cannabidiol, 2 to 40 parts of *Bletilla striata* extract, 5 to 40 parts of *Panax notoginseng* extract, 5 to 40 parts of *Corydalis yanhusuo* extract, 20-80 parts of polyvinylpyrrolidone, 1-20 parts of poloxamer, 0.01-5 parts of an antioxidant, 0.05-10 parts of polyethylene glycol and 30-120 parts of absolute ethyl alcohol. The hemostatic composition can be used as a liquid bandage for emergency treatment of the surface of a wound on the skin.

## Description

### TECHNICAL FIELD

The present invention relates to the technical field of medical devices, in particular to a hemostatic composition and use thereof as a liquid bandage.

### BACKGROUND

Cannabidiol (CDB) is one of the major Phyto cannabinoid extracts and is not found to have psycho activity or drug dependence since it does not directly activate cannabinoid type 1 receptor (CB1R) and cannabinoid type 2 receptor (CB2R). Cannabidiol has relatively high safety and good analgesic effects. Studies have shown that systemic (i.p.) injection of cannabidiol can reduce mechanical allodynia and reverse conditional place preference produced by peripheral nerve block in a rat model of incision pain. Moreover, direct injection of cannabidiol at the rostral anterior cingulate cortex produces a similar, dose-dependent effect (Genaro K, Fabris D, Arantes ALF, Zuardi AW, Crippa JAS, Prado WA. Cannabidiol Is a Potential Therapeutic for the Affective-Motivational Dimension of Incision Pain in Rats. Front Pharmacol. 2017 Jun 21; 8:391. doi: 10.3389/fphar.2017.00391. PMID: 28680401; PMCID: PMC5478794). Intramuscular injection of cannabidiol or cannabinol (CBN) can decrease mechanical sensitization and increase the mechanical threshold of masseter muscle mechanoreceptors (Wong H, Cairns BE. Cannabidiol, cannabinol and their combinations act as peripheral analgesics in a rat model of myofascial pain. Arch Oral Biol. 2019 Aug; 104:33-39. doi: 10.1016/j.archoralbio.2019.05.028. Epub 2019 May 28. PMID: 31158702). Therefore, cannabidiol can be used as an analgesic component in the field of trauma treatment.

A liquid bandage, as a novel hemostatic product for first aid, is widely used in the trauma treatment. It consists of a film material and a solvent and is sprayed or painted, so that the film-forming substance tightly adheres to the surface of the skin injury. Compared with traditional wound treatment materials, liquid bandages have the advantages of convenient carry, simple use, and stable storage. Liquid bandages maintain a right moist environment to prevent infection, scab formation or secondary mechanical injury during removal. With good mechanical properties and flexible spreadability, the film material adheres to the skin well and would not limit the local movement. Liquid bandages are waterproof and moisture-permeable, which can prevent the injury part from turning white and being infected. The transparent film material allows for better visualization of the wound recovery. The liquid bandages have wide application prospects.

CN102058900B discloses a waterproof liquid bandage for wound protection and a preparation method therefor. The liquid bandage comprises absolute ethanol, ethyl acetate, nitrocellulose, tributyl acetylcitrate, isopropyl palmitate and vitamin E acetate, and has the characteristics of physical protection, wound healing promotion, good safety and environmental friendliness, high film forming speed and, good comfort. Patent CN110964420A discloses a multifunctional antibacterial material with self-repairing property, which can be used as a liquid bandage and has long-lasting antibacterial properties. Patent CN106474537A discloses a method for preparing a liquid bandage used for war wounds. In the method, a sulfydryl-hyaluronic acid derivative, a tackifier, a humectant and a preservative are dissolved in deionized water to form a component A, and an acrylate derivative and a preservative are dissolved in deionized water and subsequently a boric acid buffer solution is added to form a component B. When using, the component A is sprayed to the wound surface at first, and subsequently the component B is sprayed to the wound surface, thus forming the liquid bandage used for war wounds. However, the aforementioned prior art does not disclose that cannabidiol is added as an analgesic component into those liquid bandages, and those liquid bandages cannot well consider the rapid film forming, rapid hemostasis, anti-inflammatory, bactericidal and analgesic effects of liquid bandages.

In view of the above, the present invention is proposed.

### SUMMARY

Aiming at the problems in the prior art, the present invention provides a cannabidiol-containing hemostatic composition and a preparation method therefor. The cannabidiol-containing hemostatic composition is used in a liquid bandage. The liquid bandage is safe and environmentally friendly, rapid in film forming, good in mechanical properties and adhesion, easy to clean, simple and convenient to use, and comfortable. Furthermore, the liquid bandage has multiple functions including rapid hemostasis, analgesic, anti-inflammatory and wound healing promotion functions.

In order to solve the above technical problems, the present invention adopts the following technical schemes:
Provided is a cannabidiol-containing hemostatic composition, which comprises, in parts by mass, 0.05 parts to 2 parts of cannabidiol, 2 parts to 40 parts of *Bletilla striata* extractextract, 5 parts to 40 parts of *Panax notoginseng* extract, 5 parts to 40 parts of *Corydalis yanhusuo* extract, 20 parts to 80 parts of polyvinylpyrrolidone, 1 part to 20 parts of poloxamer, 0.01 parts to 5 parts of an antioxidant, 0.05 parts to 10 parts of polyethylene glycol and 30 parts to 120 parts of absolute ethanol.

Cannabidiol is a natural compound with good tolerance and safety, and has good analgesic effects. Studies have shown that cannabidiol exerts positive effects in treating various diseases in preclinical and clinical trials. Cannabidiol exhibits analgesic and anti-inflammatory effects in various models, including neuropathic pain, inflammatory pain and osteoarthritis. Cannabidiol demonstrates analgesic effects in several neurogenic pain models, such as surgically induced nervous lesion (sciatic nerve injury, trigeminal infraorbital nerve injury, etc.), chronic pain caused by taking chemical substances, and neuropathic pain accompanying type 1 diabetes. Studies have shown that the free oral administration of cannabidiol-containing gelatin provides significant relief of postoperative allodynia over 3 weeks in a mouse model of sciatic nerve injury (Abraham AD, Leung EJY, Wong BA, Rivera ZMG, Kruse LC, Clark JJ, Land BB. Orally consumed cannabinoids provide long-lasting relief of allodynia in a mouse model of chronic neuropathic pain. Neuropsychopharmacology. 2020 Jun;45 (7): 1105-1114). In addition, cannabidiol has demonstrated analgesic and anti-inflammatory effects. Researches have shown that cannabidiol can relieve inflammatory pain of oral and maxillofacial region in a mouse and can exert the anti-inflammatory and analgesic effects via down-regulating inflammatory factors. In comparison to ibuprofen, cannabidiol may block ascending transduction of pain via inhibiting the activation of sp5c neurons and exert analgesic effects via regulating endocannabinoid levels. (SONG Xiankui, XIE Guanbo, WU Ning, LI Jin, HAN Xiao, GUO Hongyan. Analgesic action of cannabidiol on inflammatory pain of oral and maxillofacial region and mechanism research[J]. Journal of Practical Stomatology, 2021,37(03):307-311.). In addition, cannabidiol can reduce pain associated with arthritis. Reports have shown that transdermal cannabidiol gel significantly reduces joint swelling, limb posture scores, thickening of the synovial membrane, and pain of animals in a dose-dependent manner for 4 days in a rat model of complete Freund's adjuvant-induced monoarthritic knee joint.Besides, pro-inflammatory factors (calcitonin gene-related peptide or OX42) in spinal cord and dorsal root ganglion (TNF α) are reduced (Hammell DC, Zhang LP, Ma F, Abshire SM, McIlwrath SL, Stinchcomb AL, Westlund KN. Transdermal cannabidiol reduces inflammation and pain-related behaviours in a rat model of arthritis. Eur J Pain. 2016 Jul; 20(6):936-48. doi: 10.1002/ejp.818. Epub 2015 Oct 30. PMID: 26517407; PMCID: PMC4851925). In addition, cannabidiol may also be used in the field of pain relieving for trauma. Studies have shown that systemic (i.p.) injection of cannabidiol can reduce mechanical allodynia and reverse conditional place preference produced by peripheral nerve block in a rat model of incision pain. Moreover, direct injection of cannabidiol at the rostral anterior cingulate cortex produces a similar, dose-dependent effect (Genaro K, Fabris D, Arantes ALF, Zuardi AW, Crippa JAS, Prado WA. Cannabidiol Is a Potential Therapeutic for the Affective-Motivational Dimension of Incision Pain in Rats. Front Pharmacol. 2017 Jun 21;8:391. doi: 10.3389/fphar.2017.00391. PMID: 28680401; PMCID: PMC5478794). In conclusion, cannabidiol has good analgesic performance, and in the composition disclosed by the present invention, cannabidiol exerts excellent analgesic and anti-inflammatory effects.

*Bletilla striata* extractextract functions as a natural film-forming material, and meanwhile exerts astringent and hemostatic effects, and relieves swelling and pain. The major chemical constituents of *Bletilla striata* are *Bletilla striata* polysaccharide and volatile oil. Modern researches show that the *Bletilla striata* polysaccharide, as one of the main active ingredients of *Bletilla striata,* has remarkable pharmacological activities, such as astringent hemostasis, detumescence and promoting granulation, resisting skin chapping, antiulcer, antitumor, antibacterial, wound healing promotion, and intestinal adhesion prevention activities. Meanwhile, *Bletilla striata* has good biodegradability, biocompatibility and structural modifiability, and can be used for infection prevention and treatment of wounds.

The *Corydalis yanhusuo* extract has good analgesic effects. The main pharmacodyamic component of *Corydalis yanhusuo* is *Corydalis yanhusuo* total alkaloids. Tetrahydropalmatine in *Corydalis yanhusuo* total alkaloids is the main effective component, exerts analgesic, sedative, hypnotic and tranquilizing, and anxiolytic actions, is applicable to local anesthesia, and has effects such as relieving spasm of coronary artery, resisting arrhythmia, and resisting myocardial ischemia.

*Panax notoginseng* has good anti-inflammatory and hemostatic effects. *Panax notoginseng* contains *Panax notoginseng* total saponins, dencichine, flavones, volatile oil, amino acids, sugars and various trace elements. The main active ingredient is saponins, and the pharmacological researches in recent years show that *Panax notoginseng* saponins can dilate blood vessels, alleviate microcirculatory disturbance, reduce myocardial oxygen consumption, inhibit platelet aggregation, prolong clotting time, reduce blood lipid levels, eliminate free radicals, resist inflammation and oxidation, etc.

The present invention takes cannabidiol, *Bletilla striata* extractextract, *Corydalis yanhusuo* extract and *Panax notoginseng* extract as the functional components, which exert rapid film forming, rapid hemostasis, anti-inflammatory, bactericidal and analgesic effects, in combination with auxiliary materials.

Preferably, the composition comprises, in parts by mass, 0.05 parts to 1 part of cannabidiol, 5 parts to 40 parts of *Bletilla striata* extractextract, 5 parts to 30 parts of *Corydalis yanhusuo* extract, 5 parts to 30 parts of *Panax notoginseng* extract, 25 parts to 70 parts of polyvinylpyrrolidone, 2 parts to 16 parts of poloxamer, 0.05 parts to 5 parts of an antioxidant, 0.5 parts to 6 parts of polyethylene glycol, and 35 parts to 100 parts of absolute ethanol.

More preferably, the composition comprises, in parts by mass, 0.05 parts to 1 part of cannabidiol, 5 parts to 25 parts of *Bletilla striata* extractextract, 8 parts to 20 parts of *Corydalis yanhusuo* extract, 7 parts to 30 parts of *Panax notoginseng* extract, 25 parts to 60 parts of polyvinylpyrrolidone, 2 parts to 8 parts of poloxamer, 0.05 parts to 4 parts of an antioxidant, 0.5 parts to 4 parts of polyethylene glycol, and 35 parts to 80 parts of absolute ethanol.

Particularly preferably, the composition comprises, in parts by mass, 0.1 parts to 1 part of cannabidiol, 7 parts to 20 parts of *Bletilla striata* extractextract, 7.5 parts to 20 parts of *Corydalis yanhusuo* extract, 7 parts to 10 parts of *Panax notoginseng* extract, 25 parts to 60 parts of polyvinylpyrrolidone, 2 parts to 4 parts of poloxamer, 0.05 parts to 4 parts of an antioxidant, 0.5 parts to 4 parts of polyethylene glycol, and 35 parts to 80 parts of absolute ethanol.

In some examples of the present invention, the polyvinylpyrrolidone is polyvinylpyrrolidone K60; and/or, the poloxamer is poloxamer 407; and/or the polyethylene glycol is polyethylene glycol 6000;
the antioxidant is selected from any one or a combination of at least two of butylhydroxyanisole, dibutylhydroxytoluene, propyl gallate, tea polyphenol, phytic acid, sodium phytate, tert-butyl hydroquinone, licorice root antioxidant, phospholipid, dilauryl thiodipropionate, 4-hexylresorcinol, rosemary extract, ascorbic acid, vitamin E and bamboo leaf antioxidant; preferably, the antioxidant is dibutylhydroxytoluene.

The polysaccharide content of the *Bletilla striata* extractextract is higher than 60%;
preferably, the polysaccharide content of the *Bletilla striata* extract is higher than 70%; more preferably, the polysaccharide content of the *Bletilla striata* extract is 99%;
the purity of the cannabidiol is not less than 90%, and preferably, the purity of the cannabidiol is not less than 99%.

In some examples of the present invention, the composition comprises, in parts by mass, 25 parts to 30 parts of polyvinylpyrrolidone, 2 parts to 4 parts of poloxamer, 0.05 parts to 0.2 parts of dibutylhydroxytoluene, 0.5 parts to 2 parts of polyethylene glycol, 7 parts to 10 parts of *Bletilla striata* extract, 8 parts to 12 parts of *Corydalis yanhusuo* extract, 9 parts to 10 parts of *Panax notoginseng* extract, 36.5 parts to 40 parts of absolute ethanol, and 0.01 parts to 0.4 parts of cannabidiol.

The technical scheme of the present invention also includes use of the composition according to the present invention in the preparation of a liquid bandage. The use may be for therapeutic or non-therapeutic purposes.

The present invention also provides a preparation method for a liquid bandage comprising the aforementioned composition, which comprises the following steps: step one: weighing out a proper amount of an antioxidant and cannabidiol in proportion and dissolving in absolute ethanol; subsequently adding polyvinylpyrrolidone, poloxamer and polyethylene glycol in proportion, adding absolute ethanol, dissolving, stirring for mixing well, and allowing to stand at room temperature for 6-12 h to obtain gel matrix (a);
step two: adding *Bletilla striata* extract, *Panax notoginseng* extract and *Corydalis yanhusuo* extract in sequence for mixing with the gel matrix (a), stirring at room temperature for mixing well, allowing to stand at room temperature for 24-48 h, thus finishing the preparation.

The film forming time of the liquid bandage of the present invention is not more than 50 s under the condition of not higher than 0.1 MPa; and/or the film forming time of the liquid bandage is not more than 50 s under the condition of -20 °C to 30 °C.

The liquid bandage has at least one of the following effects: hemostatic, analgesic, anti-inflammatory, antibacterial, and promoting healing of muscle tissues, periosteum tissues and bone tissues or cell regeneration and repair.

Compared with the prior art, the composition or the liquid bandage of the present invention has quick hemostasis, analgesic and anti-inflammatory effects, and has good adhesion and mechanical properties and can protect a wound via being in direct contact with the wound surface.

### DETAILED DESCRIPTION

Unless otherwise defined, all scientific and technical terms used in the present invention have the same meaning as commonly understood by those skilled in the art to which the present invention relates.

In the present invention, the term *"Bletilla striata* extract", also known as *Bletilla striata* polysaccharide, is the viscous constituent of traditional Chinese medicine *Bletilla striata,* and is mainly composed of β-1,4-mannose, α-1,6-glucose, and β-1,4-glucose polymerized via glycosidic bonds. The *Bletilla striata* extract has pharmacological activities such as hemostatic, wound healing promotion and antibacterial activities. Meanwhile, *Bletilla striata* extract is a natural, non-toxic and non-irritant polymer material and has good water solubility, self-degradability, biocompatibility and bioadhesion. The *Bletilla striata* extract has a "two in one" property, that is, it can be used as both a main medicine of formulations and a pharmaceutical auxiliary material.

In the present invention, the term "polyvinylpyrrolidone", as a synthetic water-soluble polymer compound, has the general properties of water-soluble polymer compounds, colloid protection function, film forming property, adhesion, hygroscopicity, solubilization or aggregation function, and its most characteristic properties are excellent solubility and physiological compatibility.

In the present invention, the term "film forming time" refers to the time for the liquid bandage of the present invention to form a protective film on the wound surface.

In the present invention, the term "healing" is explained as follows, and generally, wound healing is classified as primary, secondary, and tertiary healing. Primary healing refers to the healing of a wound in which the edges are approximately closed, without cavity or dead space in the wound, such as surgical incisions without tissue defects and clean lacerations. Secondary healing refers to the healing of an open wound, with tissue destruction or tissue loss. Tertiary healing is for delayed primary closing, for example, in certain wounds, particularly infected wounds without tissue loss that are left open during the infection treatment and are surgically closed at a later stage.

In the present invention, the term "dynamic viscosity" refers to the force required for producing a unit flow velocity for unit area of fluid layer with unit distance. In the unit system, the unit of dynamic viscosity is pa.s. The calculating formula is: µ = τ/(du/dy), wherein, τ is the internal frictional resistance per unit area of the fluid flow, and du/dy is the velocity gradient. Dynamic viscosity is two indicators for assessing the viscosity of lubricating oils. The smaller the dynamic viscosity is, the better the low-temperature fluidity is; the greater the dynamic viscosity is, the poorer the low-temperature fluidity is. Embodiments of the present invention will be described in detail below with reference to examples, but those skilled in the art will appreciate that the following examples are only illustrative of the present invention and should not be construed as limiting the scope of the present invention. Experimental procedures without specified conditions in the examples are conducted according to conventional conditions or conditions recommended by the manufacturer. Reagents or instruments without specified manufacturers used herein are conventional products that are commercially available.

### Example 1

The materials for preparing the composition or the liquid bandage provided by the present invention comprise, in parts by mass, 0.3 g of cannabidiol (purity of 90%, Beijing Lanbiao Yicheng Technology Co., Ltd.), 28 g of polyvinylpyrrolidone K60, 7 g of *Bletilla striata* extract (Xian Yunhe Biotechnology Co., Ltd., polysaccharide content of 99%), 4 g of poloxamer 407, 12 g of *Corydalis yanhusuo* extract (Fufeng Sinuote Biotechnology Co., Ltd., 10:1), 10 g of *Panax notoginseng* extract (Fufeng Sinuote Biotechnology Co., Ltd., 10:1), 2 g of polyethylene glycol 6000, 0.2 g of dibutylhydroxytoluene, and 36.5 g of absolute ethanol.

The preparation method for the liquid bandage of the present invention comprises:
step one: weighing out dibutylhydroxytoluene and cannabidiol according to the aforementioned proportion and dissolving in absolute ethanol; subsequently adding PVPK60, poloxamer 407 and PEG 6000 in proportion, adding absolute ethanol, dissolving, stirring for mixing well, and allowing to stand at room temperature for 6-12 h to obtain gel matrix (a);
step two: adding *Bletilla striata* extract, *Panax notoginseng* extract and *Corydalis yanhusuo* extract in aforementioned parts by mass in sequence for mixing with the gel matrix (a), stirring at room temperature for mixing well, allowing to stand at room temperature for 24-48 h to obtain gel (b), and subpackaging to finish the preparation.

### Example 2

The preparation methods of Examples 2-4 were the same as that of Example 1, the only difference being the mass of each component. The specific data are shown in Table 1 below.

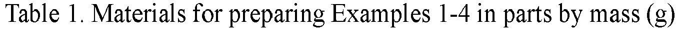

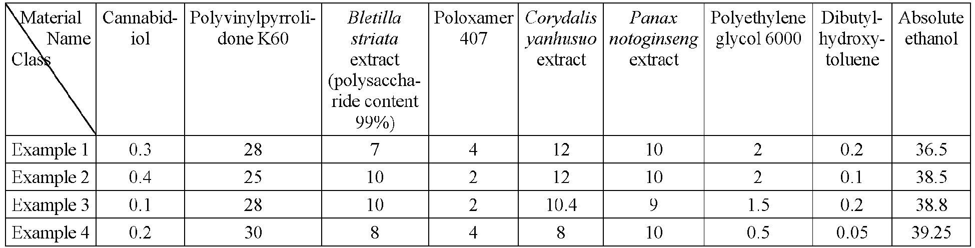

### Comparative Example 1

25 g of polyvinylpyrrolidone K60, 12 g of *Bletilla striata* extract (Xian Yunhe Biotechnology Co., Ltd., polysaccharide content of 99%), 5 g of poloxamer 407, 8 g of *Corydalis yanhusuo* extract (Fufeng Sinuote Biotechnology Co., Ltd., 10: 1), 12 g of *Panax notoginseng* extract (Fufeng Sinuote Biotechnology Co., Ltd., 10:1), 2 g of polyethylene glycol 6000, 0.2 g of dibutylhydroxytoluene, and 35.8 g of absolute ethanol. The preparation method for the liquid bandage of the present invention comprises:
step one: weighing out polyvinylpyrrolidone, poloxamer, the antioxidant and polyethylene glycol according to the aforementioned mass, dissolving in absolute ethanol under a water bath at 20 °C to 42 °C, stirring for mixing well, and allowing to stand at room temperature for 6-18 h to obtain a gel matrix (a);
step two: adding *Bletilla striata* extract, *Corydalis yanhusuo* extract and *Panax notoginseng* extract in aforementioned mass in sequence for mixing with the gel matrix (a), stirring for mixing well, allowing to stand at room temperature for 24-72 h to obtain gel (b), and subpackaging to finish the preparation.

### Experimental Example 1. Detection of Physicochemical Properties of Liquid Bandage

The liquid bandages of Examples 1-4 were taken as the test samples.
1. Appearance: All samples were brown gel-like substances, with uniform color, free of coarseness, and easy to apply.
2. Odor: All samples had the ethanol odor, and had the special odor of traditional Chinese medicines after drying.
3. Film forming time: The liquid bandages of Examples 1-4 were applied to a dry base, and the film forming time at different temperature conditions was tested. Each sample was tested 6 times, and the samples were observed, and the results were recorded. The results are shown in Table 2.

In addition, the liquid bandage of Comparative Example 1 was also subjected to the same treatment, and the film forming time thereof was measured under different temperature conditions. Each sample was tested 6 times, and the samples were observed, and the results were recorded. The results are shown in Table 3.

The normal temperature condition was as follows: room temperature 25.4 °C, RH 54%.

Note: The test samples were evenly applied to the outside of the lower part of the left arm of human body under the normal temperature condition, and under other special conditions, the samples were applied to one side of a plastic plate for testing. The results are shown in Table 2.

**Table 2 Film forming time of samples of Examples 1-4 at different temperatures**

| Class | Environmental conditions | Film forming time | | | | | | |
|---|---|---|---|---|---|---|---|---|
| | | 1 | 2 | 3 | 4 | 5 | 6 | Mean |
| Example 1 | Normal temperature and pressure | 35"14 | 42"13 | 48"05 | 34"15 | 41"32 | 32"12 | 38"55 |
| | Normal pressure at 4 °C | 31"14 | 34"16 | 28"34 | 43"32 | 37"09 | 31"07 | 34"18 |
| | Normal pressure at -20 °C | 26"11 | 28"23 | 31"34 | 28"18 | 30"03 | 34"15 | 29"47 |
| Example 2 | Normal temperature and pressure | 38"22 | 33"16 | 37"37 | 45"24 | 41"32 | 36"09 | 38"43 |
| | Normal pressure at 4 °C | 32"27 | 34"02 | 44"21 | 28"49 | 36"19 | 30"23 | 34"23 |
| | Normal pressure at -20 °C | 24"34 | 27"13 | 34"14 | 27"58 | 33"43 | 32"35 | 30"03 |
| Example 3 | Normal temperature and pressure | 42"09 | 36"03 | 39"55 | 34"25 | 45"17 | 37"08 | 39"09 |
| | Normal pressure at 4 °C | 37"26 | 31"21 | 36"07 | 37"15 | 37"13 | 29"42 | 34"50 |
| | Normal pressure at -20 °C | 34"47 | 28"09 | 32"34 | 32"04 | 27"39 | 28"37 | 30"38 |
| Example 4 | Normal temperature and pressure | 38"06 | 32"34 | 42"16 | 37"25 | 44"27 | 39"42 | 39"05 |
| | Normal pressure at 4 °C | 35"14 | 32"16 | 38"21 | 40"31 | 32"19 | 31"11 | 34"59 |
| | Normal pressure at -20 °C | 28"09 | 26"13 | 27"58 | 31"07 | 33"43 | 31"34 | 30"07 |

It can be seen from Table 2 that, the samples of Examples 1-4 had film forming time less than 50 s at normal temperature. The film forming time was different at different temperatures (normal temperature, 4 °C and -20 °C); the mean film forming time of the samples at normal temperature was 38"55, 38"43, 39"09, and 39"05, respectively, the mean film forming time of the samples at 4 °C under normal pressure was 34" 18, 34"23, 34"50, and 34"59, respectively, and the mean film forming time of the samples at -20 °C under normal pressure was 29"47, 30"03, 30"38, and 30"07, respectively. It can be concluded that the film forming time of the sample was shortened to some extent with the decrease in the ambient temperature.

**Table 3. Film forming time at different temperatures for the samples of Comparative Example 1**

| Environmental conditions | Film forming time | | | | | | |
|---|---|---|---|---|---|---|---|
| | 1 | 2 | 3 | 4 | 5 | 6 | Mean |
| Normal temperature and pressure | 45"3 | 37"24 | 47"15 | 44"15 | 34"09 | 45"47 | 42"19 |
| Normal pressure at 4 °C | 30"04 | 37"29 | 40"14 | 33"45 | 42"25 | 32"24 | 36"04 |
| Normal pressure at -20 °C | 27"37 | 32"44 | 33"43 | 28"24 | 33"43 | 37"54 | 32"19 |

It can be seen from Table 3 that, the samples of Comparative Example 1 had the film solidification time less than 60 s at normal temperature, and the film forming time was different at different temperatures (4 °C, -20 °C) and different atmospheric pressures.

In conclusion, compared with the samples of Comparative Example 1, the samples of Examples 1-4 had shorter film forming time and higher film forming speed.

4. Testing of sample pH: The samples of Examples 1-4 were weighed out accurately. Each sample was placed in a 50 mL beaker in a proportion of 1 g of sample to 9 mL of pure water, and the mixture was stirred for 5 min (if necessary, heated to 40±1 °C) to fully dissolve the sample. The temperature of the solution was adjusted to 25 °C. The electrode of a corrected acidimeter was washed with pure water and put into the sample solution. The solution was stirred for 1 min. The testing was repeated three times, wherein the error range was ±0.02. The mean value was recorded. The test results are shown in Table 4.

**Table 4. Measurement of pH of samples**

| Class | pH value | | | |
|---|---|---|---|---|
| | 1 | 2 | 3 | Mean value |
| Example 1 | 5.85 | 5.84 | 5.85 | 5.85 |
| Example 2 | 5.83 | 5.85 | 5.83 | 5.84 |
| Example 3 | 5.84 | 5.84 | 5.83 | 5.84 |
| Example 4 | 5.87 | 5.85 | 5.87 | 5.86 |

### 5. Testing of cold resistance:

Testing results: The samples of Examples 1-4 of the present invention were not solidified or layered at -20 °C.

Testing method: Two identical samples of Example 1 were placed in two pharmaceutical plastic bottles of the same specification, and the caps were tightened. One bottle was placed in a refrigerator with a previously adjusted temperature, and after 24 hours, the bottle was taken out and visually compared with the other bottle.

Judging standard: After the sample was recovered to room temperature, the appearance changes of the sample were observed.

Finally, it should be noted that, the above examples are only used to illustrate the technical schemes of the present invention, but not to limit the present invention. Although the present invention is described in detail with reference to the examples described above, it will be understood by those of ordinary skill in the art that, the technical schemes in the examples described above can still be modified, or some or all of the technical features can be equivalently replaced, and these modifications or replacements do not make the technical schemes corresponding thereto depart from the scope of the technical schemes in the examples of the present invention.

## Claims

1. A cannabidiol-containing hemostatic composition, comprising, in parts by mass, 0.05 parts to 2 parts of cannabidiol, 2 parts to 40 parts of *Bletilla striata* extract, 5 parts to 40 parts of *Panax notoginseng* extract, 5 parts to 40 parts of *Corydalis yanhusuo* extract, 20 parts to 80 parts of polyvinylpyrrolidone, 1 part to 20 parts of poloxamer, 0.01 parts to 5 parts of an antioxidant, 0.05 parts to 10 parts of polyethylene glycol and 30 parts to 120 parts of absolute ethanol.

2. The composition according to claim 1, wherein the composition comprises, in parts by mass, 0.05 parts to 1 part of cannabidiol, 5 parts to 40 parts of *Bletilla striata* extract, 5 parts to 30 parts of *Corydalis yanhusuo* extract, 5 parts to 30 parts of *Panax notoginseng* extract, 25 parts to 70 parts of polyvinylpyrrolidone, 2 parts to 16 parts of poloxamer, 0.05 parts to 5 parts of an antioxidant, 0.5 parts to 6 parts of polyethylene glycol and 35 parts to 100 parts of absolute ethanol.

3. The composition according to claim 2, wherein the composition comprises, in parts by mass, 0.05 parts to 1 part of cannabidiol, 5 parts to 25 parts of *Bletilla striata* extract, 8 parts to 20 parts of *Corydalis yanhusuo* extract, 7 parts to 30 parts of *Panax notoginseng* extract, 25 parts to 60 parts of polyvinylpyrrolidone, 2 parts to 8 parts of poloxamer, 0.05 parts to 4 parts of an antioxidant, 0.5 parts to 4 parts of polyethylene glycol and 35 parts to 80 parts of absolute ethanol.

4. The composition according to claim 3, wherein the composition comprises, in parts by mass, 0.1 parts to 1 part of cannabidiol, 7 parts to 20 parts of *Bletilla striata* extract, 7.5 parts to 20 parts of *Corydalis yanhusuo* extract, 7 parts to 10 parts of *Panax notoginseng* extract, 25 parts to 60 parts of polyvinylpyrrolidone, 2 parts to 4 parts of poloxamer, 0.05 parts to 4 parts of an antioxidant, 0.5 parts to 4 parts of polyethylene glycol and 35 parts to 80 parts of absolute ethanol.

5. The composition according to any one of claims 1-4, wherein the polyvinylpyrrolidone is polyvinylpyrrolidone K60; wherein
the poloxamer is poloxamer 407; wherein
the polyethylene glycol is polyethylene glycol 6000; wherein
the antioxidant is selected from any one or a combination of at least two of butylhydroxyanisole, dibutylhydroxytoluene, propyl gallate, tea polyphenol, phytic acid, sodium phytate, tert-butyl hydroquinone, licorice root antioxidant, phospholipid, dilauryl thiodipropionate, 4-hexylresorcinol, rosemary extract, ascorbic acid, vitamin E and bamboo leaf antioxidant; preferably, the antioxidant is dibutylhydroxytoluene; wherein the polysaccharide content of the *Bletilla striata* extract is higher than 60%; preferably, the polysaccharide content of the *Bletilla striata* extract is higher than 70%; more preferably, the polysaccharide content of the *Bletilla striata* extract is 99%; wherein
the purity of the cannabidiol is not less than 90%, and preferably, the purity of the cannabidiol is not less than 99%.

6. The composition according to claim 5, wherein the composition comprises, in parts by mass, 25 parts to 30 parts of polyvinylpyrrolidone, 2 parts to 4 parts of poloxamer, 0.05 parts to 0.2 parts of dibutylhydroxytoluene, 0.5 parts to 2 parts of polyethylene glycol, 7 parts to 10 parts of *Bletilla striata* extract, 8 parts to 12 parts of *Corydalis yanhusuo* extract, 9 parts to 10 parts of *Panax notoginseng* extract, 36.5 parts to 40 parts of absolute ethanol, and 0.01 parts to 0.4 parts of cannabidiol.

7. A preparation method for the composition according to claim 1, comprising the following steps:
step one: weighing out a proper amount of an antioxidant and cannabidiol in proportion and dissolving in absolute ethanol, subsequently adding polyvinylpyrrolidone, poloxamer and polyethylene glycol in proportion, adding absolute ethanol, dissolving, stirring for mixing well, and allowing to stand at room temperature for 6-12 h to obtain gel matrix (a);
step two: adding *Bletilla striata* extract, *Panax notoginseng* extract and *Corydalis yanhusuo* extract in sequence for mixing with the gel matrix (a), stirring at room temperature for mixing well, allowing to stand at room temperature for 24-48 h to obtain gel (b), and subpackaging to finish the preparation.

8. The composition according to claim 1, wherein the film forming time of the composition is not more than 50 s under the condition of not higher than 0.1 MPa; and/or the film forming time of the composition is not more than 50 s under the condition of - 20 °C to 30 °C.

9. Use of the composition according to any one of claims 1-8 for the preparation of a liquid bandage.

10. The use according to claim 9, wherein the liquid bandage has at least one of the following effects: hemostatic, analgesic, anti-inflammatory, antibacterial, and promoting healing of muscle tissues, periosteum tissues and bone tissues or cell regeneration and repair.
